# EUROPEAN PATENT SPECIFICATION

(11) **EP 2 371 356 B1**
(45) Date of publication and mention of the grant of the patent: **19.12.2012**
(21) Application number: 10002656.6
(22) Date of filing: 12.03.2010
(51) Int. Cl.: A61K 9/16, A61K 9/50, A61K 31/485

(54) **Multi-particle pharmaceutical formulation for colon absorption**
Pharmazeutische Mehrfachpartikelformulierung zur Kolonabsorption
Formulation pharmaceutique multiparticules pour absorption du colon

(43) Date of publication of application: 05.10.2011
(73) Proprietor: Phoeme GmbH, 8834 Schindellegi (CH)
(72) Inventor: Hermann, Lars Holger, 8834 Schindellegi (CH)
(74) Representative: Winkler, Andreas Fritz Ernst

(56) References cited:
- EP-A1- 1 203 590
- WO-A1-98/22096
- WO-A1-2004/039357
- AU-A1- 2006 200 783
- US-A- 5 643 602
- US-A1- 2002 193 445
- US-A1- 2003 152 627
- US-B1- 6 458 389

## Description

The present invention relates to multi-particulate pharmaceutical compositions which allow absorption of pharmaceutically active agent(s) in the gastrointestinal tract, including the colon. The multi-particulate pharmaceutical compositions are suitable for a once daily administration, in particular for a steady 24 hour or longer absorption of the pharmaceutically active agent(s).

### BACKGROUND OF THE INVENTION

In the treatment of diseases or ailments of the colon or rectum administration of the pharmacologically active agent to the affected site may be required. Also, absorption of a pharmacologically active agent throughout the entire or several parts of the gastrointestinal (GI) tract can be desirable for certain applications or for achieving specific serum levels of the active agent.

### Basics of Anatomy

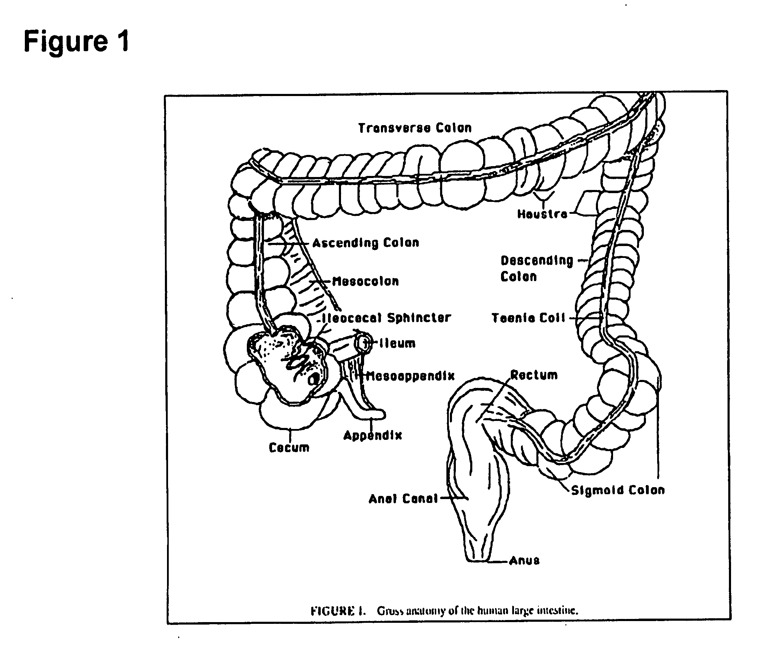

The colon, or large intestine, extends from the ileocecal junction (shared with the small intestine) to the rectum. It is composed of the cecum (with its associated vermiform appendix), ascending segment, transverse segment, descending segment, and sigmoid region (Figure 1). In the adult human, the colon is approximately 1.5 m in length and has an average diameter of about 6.5 cm, but varies in diameter from approximately 9 cm in the cecum to approximately 2 cm in the sigmoid colon. Although the colon does not have villi as observed in the small intestine, it does not demonstrate crescentric folds, termed plica semilunares, which modestly increase the internal surface area of the colon to roughly 1300 cm². Much of the colon is secured in place via retroperitoneal associations with the posterior abdominal wall. The appendix, cecum, transverse colon and sigmoid colon, however, are intraperitoneal structures that exhibit a great deal of mobility within the abdominal cavity.

Histologically, the colon is composed to four prominent layers. The outermost layer is a squamous epithelium, termed the serosa, and is found only in regions of the colon with a peritoneal surface. Deep to the serosa is the external muscular region, which consists of inner circular and outer longitudinal muscle fibers. These longitudinal fibers are localized into three flat bands, referred to as taeniae coli (Figure 1).

### Basics of Absorption

The absorption of drugs is a complex process, or more accurately, a combination or succession of complicated processes. After oral administration, the drug leaves the dosage formulation and dissolves in the aqueous digestive fluids. It reaches and crosses the gastrointestinal (GI) mucosal membrane before passing into blood. Simultaneously, a drug in solution or in solid form moves along the GI tract with the luminal content at a variable speed. The absorption process itself is influenced by the nature and surface area of the GI mucosal membrane, which varies from the stomach to the rectum, and by the physicochemical properties of the luminal content. Considerations of GI tract anatomy and the physiology of digestion led to the widely held view that drug absorption occurred primarily from the upper segments of the gut (the duodenum and jejunum).

The passage of drugs through the membrane lipid bilayer can be understood on the basis of the pH-partition hypothesis proposed by Shore and colleagues (1957). This hypothesis assumes that firstly, there is an equilibrium between the ionized and the non-ionized forms of the drug molecule, which depends on its pKa and on the pH of the medium, and secondly, only the non-ionized form is able to cross the lipid bilayer. Drug absorption is thus a purely passive process, the driving force of which is the difference in concentration, i.e. the chemical potential, across the membrane. The non-ionized hydrophobic molecule is incorporated between the long hydrocarbon chains inside the double layer by a process similar to dissolution, and is transferred into the absorptive cell. The continuous displacement of the equilibrium between the ionized and non-ionized forms, resulting from the disappearance of the latter, permits complete absorption of a drug from the GI lumen. This hypothesis for absorption bases on sufficient water molecules available for absorption. As the colon is basically a place where water molecules are absorbed, the hypotheses indicates that ionic absorption can only take place in the small intestine and in the colon ascendens. Thus colon absorption was until now based on inflammation processes changing pH and fluid in the colon like processes in colon illnesses like Colitis ulcerosa. In inflammation the colon pH decreases to acidic. Targeting colon absorption based partly until now on this phenomenon. However, pH targeting has not yet reached 24 hour absorption as the difference in pH between small intestine and colon is not sufficient.

WO 02/060415 A1 / EP 1 248 599 B1 discloses targeting colon absorption by pH, namely a multiparticulate drug form suitable for uniform release of an active pharmaceutical ingredient in the small intestine and in the large intestine, comprising at least two forms of pellets, A and B, which comprise an active pharmaceutical ingredient in the core and have different polymer coatings which determine the release of the active ingredient at different pH values, characterized in that pellet form A is provided with an inner polymer coating which enables continuous release of active ingredient, and has an outer enteric coating which rapidly dissolves above about pH 5.5, and pellet form B is provided with a polymer coating which, in the USP release test, releases less than 20% of the active ingredient at pH 6.8 in 6 hours and releases more than 50% of the active ingredient at pH 7.2 in 6 hours. This multiparticulate drug form starts to release the active agent in the duodenum at a pH of 5.5 (pellet form A) and releases in the ileum or in the colon ascendant, starting at a pH of 7.0 (pellet form B). The colon has a pH between 5.5 and 7.0. A colon targeting is not sufficient if only pH values are taken in consideration. The pH in the colon normally varies between 5.5 and 7 (slightly acidic to neutral). Thus a pH dependent targeting does not suffice as these pH values are also found in the small intestine.

WO 2006/102446 also discloses targeting colon absorption by pH, namely a multi-particulate, modified-release pharmaceutical composition for the oral administration of an active ingredient to the colon, wherein said particles comprise: (a) a core comprising an active ingredient or a pharmaceutically acceptable salt or ester thereof, and optionally one or more excipients; (b) a first coating applied to the surface of the core, wherein said first coating is insoluble in gastric juice and in intestinal juice below pH 7, but soluble in colonic intestinal juice; and (c) a second coating applied to the surface of the first coating.

In US 6,039,975 a delivery system for targeting drugs to the colon is disclosed. The delivery system is a tablet comprised of three parts: (1) an enteric coating to prevent penetration of gastric fluid into the delivery system, thereby preventing any drug release in the stomach; (2) an erodible polymer layer which is exposed and gradually erodes during transit through the upper intestinal tract, and (3) a core, which is a conventional tablet or beadlet containing an active ingredient(s), which readily disintegrates and subsequently releases the drug to the target site, the colon, after erosion of the erodible polymer layer. The erodible polymer layer prevents drug release in the upper portion of the intestinal tract for 4-6 hours after gastric emptying, representing the amount of time needed for the delivery system to reach the colon.

There is a need in the art for pharmaceutical compositions that allow controlled release of pharmaceutically active agent(s) and achieve desired serum levels of the pharmaceutically active agent(s) via absorption throughout the gastrointestinal tract, including the colon.

### SUMMARY OF THE INVENTION

According to the present invention this object is solved by providing a multi-particulate pharmaceutical composition or formulation.

Said multi-particulate pharmaceutical composition or formulation has a particle size of between 2.2 and 4 mm, preferably 2.5 to 3.1 mm and most preferably about 2.8 mm.

Each particle of said multi-particulate pharmaceutical composition or formulation comprises:
(a) a core containing or carrying at least one pharmaceutically active agent; and
(b) at least one layer showing adhesion and/or being crushable in the colon comprising (i) one or more poly(meth)acrylates, and (ii) non-porous inert lubricant, selected from the group consisting of stearates, kaolin, pharmaglass, talcum and mixtures thereof.

According to the present invention this object is furthermore solved by providing the composition for a once daily administration.

According to the present invention this object is furthermore solved by providing the composition for a once daily administration for obtaining a steady 24 hour or longer absorption of the at least one pharmaceutically active agent.

### DESCRIPTION OF THE PREFERRED EMBODIMENTS OF THE INVENTION

Before the present invention is described in more detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art. For the purpose of the present invention, all references cited herein are incorporated by reference in their entireties.

### Multi-particulate pharmaceutical compositions

As outlined above, the present invention provides multi-particulate pharmaceutical compositions or formulations.

The multi-particulate pharmaceutical compositions or formulations of the invention are preferably for oral administration.

The present invention originates from controlled release pharmaceutical forms for oral administration. This type of pharmaceutical form is intended for more or longer- lasting release of active compound, usually during intestinal passage. It is attempted to achieve by means of appropriate formulation of the pharmaceutical form, that, after an initial increase of the concentration of the active compound in the blood or serum level, the blood or serum level shall remain in the therapeutically optimal range as long as possible. Especially too high blood level concentrations of the active compound, which may have toxic effects, should be avoided.

Each particle of said multi-particulate pharmaceutical composition or formulation comprises:
(a) a core containing or carrying at least one pharmaceutically active agent; and
(b) at least one layer showing adhesion and/or being crushable in the colon comprising (i) one or more poly(meth)acrylates, and (ii) non-porous inert lubricant, selected from the group consisting of stearates, kaolin, pharmaglass, talcum and mixtures thereof.

The multi-particulate pharmaceutical compositions or formulations according to the invention are suitable for
- substance / active agent absorption in stomach, small intestine and full colon (absorption beginning in the stomach and including at least colon transversum and even descendens or ampulla recti);
- obtaining a steady 24 hour or longer absorption of the pharmaceutically active agent by using all parts of the GI tract for absorption including the colon.

"Colon absorption" according to this invention refers to a sufficient absorption documented by serum levels in an amount of more than 10 % of the whole amount absorbed in stomach, small intestine and colon.

A number of approaches have been explored to improve or enhance drug absorption from the GI tract while minimizing the variability of delivery. Most studies to date have been directed at the small intestine, rather than the colon; however, with the current interest in colonic delivery, more studies have focused on this area of the GI tract. In general, approaches to enhance drug absorption have attempted to reduce premature metabolism and chemical destruction of the drug, to lengthen the residence time of the drug in the colon, and to increase the rate of drug transport across the intestinal mucosa by altering rate-limiting barriers. The use of chemical enhancers, prodrugs, enzyme inhibitors, drug modifications, bioadhesives, and particulates have all been described (Friend, 1992). The effect of the formulations of the present invention is mainly based upon particle size but is also the adhesive /crushable layer and specific polaycrylates.

Surprisingly, the inventors have found in the present study in humans that a combination of a pellet formulation with particles/pellets having a size of about 3 mm with an adhesive coating / crushable coating preferably comprising a polymer mix with a special polymer opening at preferably pH > 7.4 enables a serum level which is proof of a colon absorption for a morphine formulation which shows a serum profile that starts less than 20 min after ingestion of the medication and keeps releasing for at least 22 hours. This is proof of substance absorption beginning in the stomach and including at least colon transversum and even descendens or ampulla recti.

### Particle size

The multi-particulate pharmaceutical composition or formulation according to the invention has a particle size of between 2.2 and 4 mm.

Preferably the particle size is 2.5 to 3.1 mm and most preferably about 2.8 mm.

The particles are preferably pellets, mini tablets, or any other particles used for pharmaceutical purposes in this size for oral use.

The invention is directed at a formulation with a particle or pellet size larger than 2.2 mm up to 4 mm.

Classical formulations have postulated that particles/pellets larger than 2 mm are held back by the gastric emptying process of the pylorus. The serum absorption of morphine in the present study does document surprisingly that a formulation with particles of more than 2.5 mm particle size is permitted through the gastric pylorus. Explanations could be that it seems to have a "cloud effect". Particles are parted into a cloud of absorption and enable a higher absorption which does not stop at the classical absorption targets of Jejunum and Ileum. The measured serum levels prove the unexpected effect of 24 hour absorption in contrast to the classical absorption of average of a maximum of 12 to 14 hours. It was surprisingly shown in the present study that the above postulation of 2 mm is not valid. Instead, it was documented that particles sized larger than 2.2 mm are having a unique effect. In the formulation according to the invention pellets between 2.2 and 4 mm with an average of 2.8 mm are found. Another explanation is that particles of 2.2 mm up to 4 mm are crushed through motility of the colonic muscles.

### (a) Core

The core (a) contains or carries at least one pharmaceutically active agent.

Preferably, the core carries or contains the at least one pharmaceutically active agent in the form of a coating or in the form of the pharmaceutically active agent incorporated in the core.

Preferably, the pharmaceutically active agent has a half life of less than or about 24 hours.

Sustained release medications do have the aim of prolonging a half life time. For example, morphine has a half life of about 4 hours. Thus oral intake would have to be approximately six times a day. Any substance of a half life of less than the time until the last resorption is terminated can profit from this invention, i.e. the present invention will be advantageous for such substances / pharmaceutically active agents. Absorption takes place until a maximum of 24 hours. Thus any medication with a half life time of less than 24 hours is relevant for this invention.

Preferably, the pharmaceutically active agent is an opioid.

Besides the pharmaceutical active ingredient, the core may comprise further components, such as pharmaceutical excipients: binders such as cellulose and derivatives thereof, polyvinylpyrrolidone (PVP), humectants, disintegration promoters, lubricants, disintegrants, starch and derivatives thereof, sugar solubilizers or others.

### (b) Adhesion /crushable layer(s)

The at least one layer (b) showing adhesion and/or being crushable in the colon comprises (i) one or more poly(meth)acrylates, and (ii) non-porous inert lubricant, selected from the group consisting of stearates, kaolin, pharmaglass, talcum and mixtures thereof.

This (at least one) layer (b) serves for
- adhesion of the pharmaceutical composition in the GI tract, in particular adhesion to the intestinal villi, and/or
- (mechanical) destruction/crushing of the pharmaceutical composition in the GI tract, in particular crushing in the intestinal villi.

The adhesive or crushable layer(s) are non specific layer(s) which mainly serves to enlarge pellets and specify dissolution.

### (i) Polymers

Preferably, the one or more poly(meth)acrylates (i) are a polymer mixture comprising at least one water insoluble poly(meth)acrylate and at least one water soluble poly(meth)acrylate. Preferably, the at least one water insoluble poly(meth)acrylate is a copolymer of ethyl acrylate and methyl methacrylate; and preferably the at least one water soluble poly(meth)acrylate is a copolymer of methyl acrylate, methyl methacrylate and methacrylic acid.

Preferably, the polymer mixture of (i) comprises
- 40 - 95 % by weight (preferably 50 - 90 % by weight, more preferably 60 - 80 % by weight, even more preferably about 70 % by weight, more preferably about 67 % by weight), based on the dry weight of the polymer mixture, of at least one water insoluble poly(meth)acrylate (copolymer), and
- 5 - 60 % by weight (preferably 10 - 40 % by weight, more preferably 20 - 30 % by weight, more preferably about 23 % by weight), based on the dry weight of the polymer mixture, of at least one water soluble poly(meth)acrylate (copolymer).

Water soluble and insoluble poly(meth)acrylates or copolymers thereof are known in the art, such as disclosed in WO 2009/036812 (by Evonik Röhm GmbH, Germany).

WO 2009/036812 discloses pH-dependent controlled release pharmaceutical opiod compositions with resistance against the influence of ethanol, comprising a core, comprising at least one pharmaceutical active ingredient, which is an opioid, wherein the core is coated at least by one coating layer, controlling the release of the pharmaceutical composition, wherein the coating layer comprises a polymer mixture of i) 40 - 95 % by weight, based on dry weight of the polymer mixture, of at least one water insoluble essentially neutral vinyl polymer, and ii) 5 - 60 % by weight, based on dry weight of the polymer mixture, of at least one anionic polymer or copolymer, which is insoluble in a buffered medium below pH 4.0 and soluble at least in the range from pH 7.0 - 8.0, characterized in that the coating layer further contains 110 to 250 % by weight, calculated on dry weight of the polymer mixture, of a non-porous inert lubricant and the coating layer is present in an amount of at least 60 % by weight calculated on the weight of core.

Preferred water soluble poly(meth)acrylates are (meth)acrylate copolymers consisting of 10 to 30% by weight methyl methacrylate, 50 to 70% by weight methyl acrylate and 5 to 15% by weight methacrylic acid (Eudragit® FS type, such as Eudragit® FS 30 D, Evonik Röhm GmbH, Germany). The pH at the start of the specific active ingredient release is pH 7.0. Eudragit® FS is a copolymer polymerized out of 25% by weight methyl meth- acrylate, 65% by weight methyl acrylate and 10% by weight methacrylic acid. Eudragit® FS 30 D is a dispersion comprising 30% by weight Eudragit® FS.

Eudragit® FS 30 D releases in the Ileum or in the colon ascendant, starting at a pH of 7.0.

Preferred water insoluble poly(meth)acrylates are neutral or virtually neutral (meth)acrylate copolymers composed of 20 to 40% by weight of ethyl acrylate, 60 to 80% by weight of methyl methacrylate and 0 to less than 5% by weight, preferably 0 to 2 or 0.05 to 1 % by weight of acrylic acid or methacrylic acid (Eudragit® NE or Eudragit® NM type, such as Eudragit® NE 30 D, Evonik Röhm GmbH, Germany)

Eudragit® NE and Eudragit® NM are copolymers composed of free-radically polymerized units of 30% by weight of ethyl acrylate and 70% by weight of methyl methacrylate.

### (ii) Lubricant

"Lubricants" (sometimes also called glidants) are pharmaceutically acceptable substances which help in preventing agglomeration of polymers during the coating process.

Porous lubricants like silica powders are not suitable for the purposes of the present invention. "Inert" refers to that the lubricant does (normally) not chemically interact with other substances and is not soluble or only poorly soluble in water. Not soluble or only poorly soluble means more than 10 parts by weight of solvent required per 1 part by weight of solute.

The non-porous inert lubricant (ii) is selected from the group consisting of stearates, kaolin, pharmaglass, talcum and mixtures thereof.

In a preferred embodiment, the non-porous inert lubricant is a stearate, preferably Mg stearate, or talcum.

In this embodiment, the composition comprises 5 to 30 %, preferably 8 to 15 %, more preferably about 9 % by weight, based on the dry weight of the polymer mixture of (i), of Mg stearate.

In a preferred embodiment, the non-porous inert lubricant is talcum.

In this embodiment, the composition comprises 100 to 300 %, preferably 150 to 250 %, more preferably about 200 % by weight, based on the dry weight of the polymer mixture of (i), of talcum.

### (c) Further layers or coats

In a preferred embodiment, the compositions of the invention further comprise a *barrier layer.*

The barrier layer preferably comprises at least one poly(meth)acrylate and a non-porous inert lubricant, selected from the group consisting of stearates, kaolin, pharmaglass, talcum and mixtures thereof.

Preferably, the at least one poly(meth)acrylate is a water soluble poly(meth)acrylate or copolymer thereof, preferably as described above (such as copolymer of methyl acrylate, methyl methacrylate and methacrylic acid).

Preferably, the non-porous inert lubricant is a stearate.

In a preferred embodiment, the compositions of the invention further comprise a *top coat.*

The top coat preferably contains at least one pharmaceutically active agent.

Preferably, the top coat contains the pharmaceutically active agent or at least one of the pharmaceutically active agents of the core (a).

This embodiment allows a first or fast release of the active agent, such as in an initial burst, before the release of the active agent from the core (a) starts.

Exemplary further components of the top coat are shown in Examples 1 and 2.

### Use of the pharmaceutical compositions

As outlined above, the present invention provides the compositions for a once daily administration.

As outlined above, the present invention provides the compositions for a once daily administration for obtaining a steady 24 hour or longer absorption of the at least one pharmaceutically active agent.

The term "steady absorption" refers to an absorption which results in a blood or serum level of the active agent with deviation of less than 25 % from the medium concentration. Furthermore, the term "steady absorption" preferably refers to documentable/measurable absorption process from the gut resulting in an increase not a decrease in serum levels of the active agent 14 hours after administration.

The basic idea about retardation for a once daily medication is the provision of sufficient molecules in the blood to ensure that substances with a half life of less than 24 hours can be taken once a day. The ideal medication would provide a 24 hour flat line of a sufficient serum level. The less difference from the median serum levels the more of the desired effect is delivered with less side effects arising from to much substance.

Current morphine sustained release medications of opioids or other substances with the necessity for sustained release formulations show an initial peak which is reduced per hour with no further absorption after 8 hours (ileum until possible colon ascendens passage). However, this first peak induces negative effects of opioid medications which can culminate in parenteral application like euphorisation, sedation, breath depression etc.

One major positive effect of the absorption accomplished by the invention is a constant absorption with a constant blood level. Thus no peaks or valleys of documented absorption can be demonstrated. Clinically a 24 hour constant blood level, reachable by colon absorption, with deviation of less than 25 % from the medium concentration is a huge step for opioid indications of pain and drug substitution or other substances with the necessity for sustained release formulations. A once daily prescription with a serum level with as few deviation form the median as possible offers several advantages: High peaks show added problems of side effects and even leaving the therapeutic index of the substance defined as the ratio of desired effect to toxic effect. As opioids are compounds with a narrow therapeutic index and exert their desired effects at a dose close to their toxic dose, a stable serum level for a long period is the desired application with these medications. Toxic or side effects are breath depression, intoxication, euphorisation and others. Avoiding these side effects or toxic effects allows better use of the potentially dangerous opioid medications or other substances with the necessity for sustained release formulations.

### Colon transit time and absorption

In general, though, mouth-to-colon transit in humans has been estimated to be 8 to 10 h, with a transit time through the small intestine of 2 to 4 h. In another study, Hardy and co-workers (Hardy et al., 1985) suggest optimal delivery to the colon would use the average time of 4 h transit through the small intestine and stomach, but 10 h for release during transit through the ascending and transverse colon. Although it has been argued that multiple-unit, controlled-release dosage forms offer substantial advantages over single-unit dosage forms for delivery to the colon, recent studies have suggested single and multiple dosages appear to be comparable and demonstrate transit times consistent with previous studies (Hardy et al., 1985). The major site of GI transit variability appears to be the stomach, where the presence of food and the size of the dosage form can affect gastric residence time. Dosage form density has also been suggested to affect gastric residence time. Once in the colon, transit through the left colon (cecum to mid-transverse colon) requires approximately 11 h, transit through the right colon (mid-transverse to sigmoid colon) also takes about 11 h, and residence time in the sigmoid colon averages nearly 12.5 h. In another study (Coupe et al., 1991) these transit times were measured to average approximately 7 h, 9 h, and 18.5 h for the three regions of the colon described above. In total, transit time through the colon consistently requires approximately 35 to 36 h.

Investigations (Malagelada, 1977) suggest a screening effect of the pylorus for solid particles larger than approximately 2 mm. This process is more efficient shortly after food intake but is never 100 % efficient so that larger solids may occasionally be emptied soon after a meal. The retention of objects larger than 2 mm may be of considerable significance when enteric coated or sustained-release tablets are administered with food.

### Evaluation of the compositions in a human study

A number of models have been used to study the physical and enzymatic barriers to colonic absorption. Exposure of drug species to colonic cell homogenates, intact cells, or isolated epithelia have been used to investigate enzymatic barriers to transport. Isolated colonic epithelia or cultured colonic cells have been used to assess the physical barriers to the transport of drug molecules. *But in situ and in vivo* methods have been used to evaluate total transport potential, particularly from complex formulations of numerous drug entities and model compounds. It is difficult to know, a priori, the essential barrier(s) to the transport of a specific molecule. Isolated cell systems, although better characterized, may not provide the unique route of uptake that *occurs in vivo.* Although *in vivo* systems are often labor intensive, difficult to standardize, and usually present the greatest differences between human and other species, they provide a glimpse of the total transport potential for a given drug not observable in many *in vitro* systems. Negative results in an *in vivo* study, however, can often lead to inconclusive results due to the complexity of the model.

This shows that the effects of the pharmaceutical composition of the invention the inventors have shown in a human study are fully unexpected and could not be anticipated with *in vitro* and *in vivo* methods.

Each approach can provide a unique set of information. Interpretation of results from each approach, however, must be recognized for its limitations as well as its advantages. Therefore, cumulative studies employing cell culture, *in vitro* systems, and *in vivo* methods may be necessary to yield the depth of understanding required for successful colonic drug delivery. Ultimately, human subjects must be used for studies. Thus, the human study was the only possibility to show the effect the inventors postulate and could not be expected by *in vivo* dissolution profiles which differ totally from the reached results.

The following examples and drawings illustrate the different aspects of the present invention without, however, limiting the same thereto.

### BRIEF DESCRIPTION OF THE DRAWINGS

**Figure 1****.** *Gross anatomy of the human large intestine.*
**Figure 2****.** *Comparison of typical* / *classical serum levels for currently known medications and serum levels of a composition according to the invention.*
(shown for morphine).
Circles: currently known medication ("registered medication")
Triangles: composition according to the invention ("new medication").

### EXAMPLES

"%" in this context means "% by weight", unless indicated otherwise.

### Example 1

Pellets were made that contain (a) particle cores that are coated with the pharmaceutically active agent ("Particles with active agent") and (b) a layer showing adhesion and/or being crushable in the colon comprising (i) two types of poly(meth)acrylate (the water soluble Eudragit® FS 30 D and the water insoluble Eudragit® NE 30 D) and (ii) Mg stearate as lubricant ("poly(meth)acrylate layer").

Morphine sulfate was used as a model drug.

The pellets furthermore contain several separation layers and a barrier layer.

The pellets furthermore contain a top coat, which also comprises the pharmaceutically active agent.

All layers (except the top coat and the last separation layer) are applied by spraying.

### (1) Particle cores

| Component | Amount [g] | | % |
|---|---|---|---|
| | | | |
| Sugar starch | | 2500 | 38.64 |
| pellets | | | |

| *Spray for coating with the active agent* | | | |
|---|---|---|---|
| *Active agent* | 100 % amount | Solids [g] | |
| *coating* | [g] | | |
| Morphine sulfate | 937.5 | 937.5 | 14.49 |
| pentahydrate | | | |
| Titan dioxide | 93.8 | 93.8 | 1.45 |
| Kollidon K 25 | 281.3 | 281.3 | 4.35 |
| Water, purified | 2187.4 | 0 | |
| | 3500 | 1312.6 | |
| | | | |
| *Separation layer* | | | |
| Aerosil 200 | 13.8 | 13.8 | 0.21 |
| Water, purified | 261.3 | 0 | |
| | 275.1 | 13.8 | |
| | | | |
| Particles with | | 3826.4 | |
| active agent | | | |

### (2) Particles with active agent and barrier layer

| Component | Amount [g] | | % |
|---|---|---|---|
| | | | |
| Particles with active | | 1500 | |
| agent (1) | | | |
| *Barrier layer* | 100 % amount [g] | Solids [g] | |
| Methyl acrylate, | 300 | 90 | 3.55 |
| methyl methacrylate | | | |
| and methacrylic | | | |
| acid copolymer [2] | | | |
| Mg stearate | 9 | 9 | 0.35 |
| Water, purified | 186 | 0 | |
| | 495 | 99 | |
| | | | |
| Particles with | | 1599 | |
| barrier layer | | | |

### (3) Particles with active agent, barrier layer and poly(meth)acrylate layer

| Component | Amount [g] | | % | |
|---|---|---|---|---|
| | | | | |
| Particles with | | 1599.0 | | |
| barrier layer (2) | | | | |

| *Spray for applying the poly(meth)acrylate layer* | | | | |
|---|---|---|---|---|
| *Poly(meth)acrylate* | 100 % amount [g] | Solids [g] | Solids [%] | |
| *layer* | | | | |
| Ethyl acrylate and | 1700 | 510 | 56.7 | 20.11 |
| methyl methacrylate | | | | |
| copolymer [3] | | | | |
| Methyl acrylate, | 300 | 90 | 10 | 3.55 |
| methyl methacrylate | | | | |
| and methacrylic | | | | |
| acid copolymer [4] | | | | |
| Mg stearate | 60 | 60 | 6.7 | 2.37 |
| Titan dioxide | 60 | 60 | 6.7 | 2.37 |
| Polysorbat 80 | 60 | 60 | 6.7 | 2.37 |
| HPMC | 120 | 120 | 13.3 | 4.73 |
| Water, purified | 2200 | 0 | 0 | |
| | 4500 | 900 | 100 | |
| | | | | |
| *Separation layer* | | | | |
| Aerosil 200 | 7.5 | 7.5 | | 0.3 |
| Water, purified | 142.5 | 0 | | |
| | 150 | 7.5 | | |

### (4) Particles with active agent, barrier layer, poly(meth)acrylate layer and top coat

| Component | Amount [g] | | % |
|---|---|---|---|
| | | | |
| Particles (3) | | | |
| *Top coat* | 100 % amount [g] | Solids [g] | |
| PVP (Kollidon 25) | 6.27 | 5.1 | 0.20 |
| Morphine sulfate | 18.7 | 17.1 | 0.67 |
| pentahydrate | | | |
| Water, purified | 225.5 | 0 | |
| | 250.47 | 22.2 | |
| | | | |
| *Separation layer* | | | |
| Aerosil 200 | 7.5 | 7.5 | 0.3 |
| Water, purified | 142.5 | 0 | |
| | 150 | 7.5 | |
| **Final weight** | | **2536.2** | 100 % |

[2] As a component of the dry substance of Eudragit® FS 30 D
[3] As a component of the dry substance of "Polyacrylate Dispersion 30 Per Cent (Ph.Eur.) (Eudragit® NE 30 D)
[4] As a component of the dry substance of Eudragit® FS 30 D

The final weight is 2536.2 g.

The content of active agent (Morphine sulfate pentahydrate) is 15.16 %.
The amount of polymers coated is 40 %.

The sizes of the finished particles are in the range from 2.1 (minimum) to 3.5 mm (maximum), with an average of 2.8 mm.

### Example 2

| Component | Amount [mg] |
|---|---|
| | |
| ***Pellets with drug substance (core)*** | |
| Morphine sulphate pentahydrate | 191.05 |
| Sucrose [1] | 436.86 |
| Maize starch | 72.6 |
| Titanium dioxide (E 171) | 19.11 |
| Povidone (K 25) | 57.32 |
| ***Separation layer I*** | |
| Silica, colloidal anhydrous | 2.81 |
| ***Modified release film 1 (barrier layer)*** | |
| Methyl acrylate, methyl | 44.45 |
| methacrylate and methacrylic acid | |
| copolymer [2] | |
| Polysorbate 80 | 1.87 |
| Sodium laurilsulfate | 0.47 |
| Talc | 9.36 |
| ***Modified release film 2 (poly(meth)acrylate layer)*** | |
| Ethyl acrylate and methyl | 251.86 |
| methacrylate copolymer [3] | |
| Nonoxynol 100 | 13.26 |
| Methyl acrylate, methyl | 44.45 |
| methacrylate and methacrylic acid | |
| copolymer [4] | |
| Polysorbate 80 | 1.87 |
| Sodium laurilsulfate | 0.47 |
| Talc | 623.8 |
| Titanium dioxide | 31.19 |
| Polysorbate 80 | 31.19 |
| Hypromellose 6 mPa·s | 62.38 |
| ***Separation layer 2*** | |
| Silica, colloidal anhydrous | 3.9 |
| *Top-coating layer (top coat)* | |
| Morphine sulphate pentahydrate | 8.89 |
| Povidone (K 25) | 2.65 |
| ***Separation layer 3*** | |
| Silica, colloidal anhydrous | 3.9 |
| **Sum** | **1915.71** |

| | |
|---|---|
| [1] As a component of sugar spheres (Ph.Eur.) [2] As a component of the dry substance of Eudragit® FS 30 D [3] As a component of the dry substance of "Polyacrylate Dispersion 30 Per Cent (Ph.Eur.) (Eudragit® NE 30 D) [4] As a component of the dry substance of Eudragit® FS 30 D | |

The sizes of the finished particles are in the range from 2.1 (minimum) to 3.5 mm (maximum), with an average of 2.8 mm.

### Example 3

(a) Typical / Classical serum levels for currently known medications:
Basing on the following GI transit time:

| | *According to Coupe et al., 1991:* | *According to Hardy et al., 1985:* |
|---|---|---|
| Mouth to end of small intestines | 0 - 4 h | 0 - 8 h |
| Colon ascendens | 4 -10 h | 8 - 13 h |
| Colon transverses | 10 - 15 h | 13 - 18 h |
| Colon descendens | 15 - 26 h | 18 - 24 h |

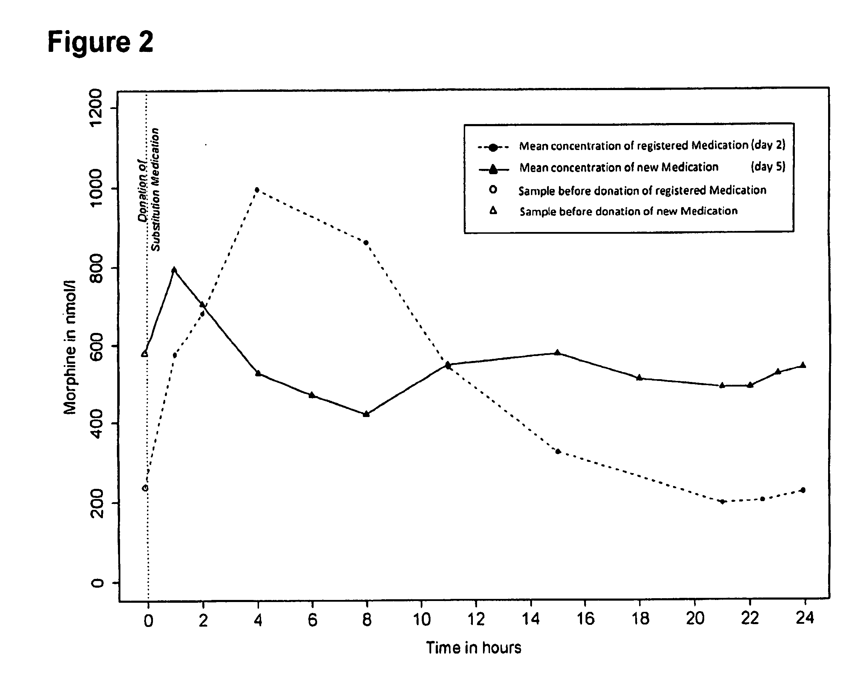

See Figure 2 (circles), showing morphine serum levels:
Absorptions maximum small intestine after 2 to 4 hours
No added absorption after 8 to 10 hours
(b) Using a composition according to the invention, in particular of Example 1.
See Figure 2 (triangles), showing morphine serum levels:
First Absorption:
   Absorption stomach 0.5 hours
   Absorption hour 1 - 5 / 8 duodenum jejunum ileum
   Add absorption first time fully documented absorption in hour 8 to 23 as transit time colon ascendens, transvers and descends and rectum.

Half life about 4 hour no sufficient explanation for serum levels

Glucoronidation and re - resorption not sufficient explanation

Dissolution profile *in vitro* only 18 hours dissolution

New totally unexpected results were documented in a study in November 2009 for 10 patients, which is a methodological research fully documented under the Austrian "Arzneimittelgesetz" and fully approved.

### REFERENCES

Coupe A.J, Davis S.S., Wilding I.R., Variation in gastrointestinal transit of pharmaceutical dosage forms in healthy subjects, Pharm. Res., 8, 360, 1991.
Friend D. R., Oral colon-specific drug delivery, CRC Press Inc., 1992.
Hardy J.G., Wilson C. G., Wood E. (1985). Drug Delivery to the proximal colon, J Pharm Pharmacol., 12, 874-7.
Malagelada J.R. (1977). Quantification of gastric solid-liquid discrimination during digestion of ordinary meals, Gastroenterology, 72, 1264-1267.
Shore P.A., Brodie B. B. & Hogben, C. A.M. (1957). The gastric secretion of drugs : a pH partition hypothesis. JPharmac. exp. Ther., 119, 361-369.

## Claims

1. Multi-particulate pharmaceutical composition, the particles having a particle size of between 2.2 and 4 mm, preferably 2.5 to 3.1 mm and most preferably about 2.8 mm, each particle comprising:
(a) a core containing or carrying at least one pharmaceutically active agent;
(b) at least one layer showing adhesion and/or being crushable in the colon comprising (i) one or more poly(meth)acrylates, and (ii) non-porous inert lubricant, selected from the group consisting of stearates, kaolin, pharmaglass, talcum and mixtures thereof; and
(c) a top coat containing at least one pharmaceutically active agent,
wherein the one or more poly(meth)acrylates (i) are a polymer mixture comprising 40 - 95 % by weight, based on the dry weight of the polymer mixture, of at least one water insoluble poly(meth)acrylate and 5 - 60 % by weight, based on the dry weight of the polymer mixture, of at least one water soluble poly(meth)acrylate.

2. The composition of claim 1, wherein the top coat contains the pharmaceutically active agent or at least one of the pharmaceutically active agents of the core (a).

3. The composition of claim 1 or 2, wherein the pharmaceutically active agent has a half life of less than or about 24 hours.

4. The composition of any of claims 1 to 3, wherein the pharmaceutically active agent is an opioid.

5. The composition of any of the preceding claims, wherein the polymer content is about 40 % by weight of the total weight of the composition.

6. The composition of any of the preceding claims, wherein the at least one water insoluble poly(meth)acrylate is a copolymer of ethyl acrylate and methyl acrylate; and wherein the at least one water soluble poly(meth)acrylate is a copolymer of methyl acrylate, methyl methacrylate and methacrylic acid.

7. The composition of any of the preceding claims, wherein the polymer mixture (i) comprises
50 - 90 % by weight, preferably 60 - 80 % by weight, more preferably about 70 % by weight, more preferably about 67 % by weight, based on the dry weight of the polymer mixture, of at least one water insoluble poly(meth)acrylate, and 10 - 40 % by weight, preferably 20 - 30 % by weight, more preferably about 23 % by weight, based on the dry weight of the polymer mixture, of at least one water soluble poly(meth)acrylate

8. The composition of any of the preceding claims, wherein the non-porous inert lubricant is stearate, preferably Mg stearate, or talcum.

9. The composition of claim 8, comprising 5 to 30 %, preferably 8 to 15 %, more preferably about 9 % by weight, based on the dry weight of the polymer mixture of (i), of Mg stearate.

10. The composition of claim 8, comprising 100 to 300 %, preferably 150 to 250 %, more preferably about 200 % by weight, based on the dry weight of the polymer mixture of (i), of talcum.

11. The composition of any of the preceding claims, further comprising a barrier layer comprising at least one poly(meth)acrylate and a non-porous lubricant, selected from the group consisting of stearates, kaolin, pharmaglass, talcum and mixtures thereof,
wherein the at least one poly(meth)acrylate is preferably a water soluble poly(meth)acrylate and the non-porous lubricant is preferably a stearate.

12. Composition of any of claims 1 to 11 for a once daily administration.

13. Composition of any of claims 1 to 11 for a once daily administration for obtaining a steady 24 hour or longer absorption of the at least one pharmaceutically active agent.

## Patentansprüche

1. Pharmazeutische Mehrfachpartikelzusammensetzung, wobei die Partikel eine Partikelgröße von zwischen 2,2 und 4 mm, vorzugsweise 2,5 bis 3,1 mm und am bevorzugtesten etwa 2,8 mm aufweisen, wobei jedes Partikel:
(a) einen Kern, der wenigstens einen pharmazeutischen Wirkstoff enthält oder trägt;
(b) wenigstens eine Schicht, die im Colon Adhäsion zeigt und/oder zerkleinerbar ist, die (i) ein oder mehrere Poly(meth)acrylate und (ii) nicht-poröses inertes Gleitmittel, das ausgewählt aus der Gruppe, bestehend aus Stearaten, Kaolin, Pharmaglas, Talkum und Mischungen davon, umfasst; und
(c) einen Decküberzug, der wenigstens einen pharmazeutischen Wirkstoff enthält,
umfasst,
wobei das eine oder die mehreren Poly(meth)acrylate (i) eine Polymermischung sind, die 40 - 95 Gew. - %, bezogen auf das Trockengewicht der Polymermischung, an wenigstens einem wasserunlöslichen Poly(meth)acrylat und 5 - 60 Gew. - %, bezogen auf das Trockengewicht der Polymermischung, an wenigstens einem wasserlöslichen Poly(meth)acrylat umfasst.

2. Zusammensetzung nach Anspruch 1, wobei der Decküberzug einen pharmazeutischen Wirkstoff oder wenigstens einen der pharmazeutischen Wirkstoffe des Kerns (a) enthält.

3. Zusammensetzung nach Anspruch 1 oder 2, wobei der pharmazeutische Wirkstoff eine Halbwertszeit von weniger als oder etwa 24 Stunden aufweist.

4. Zusammensetzung nach einem der Ansprüche 1 bis 3, wobei der pharmazeutische Wirkstoff ein Opioid ist.

5. Zusammensetzung nach einem der vorangehenden Ansprüche, wobei der Polymergehalt bei etwa 40 Gew. - % des Gesamtgewichts der Zusammensetzung liegt.

6. Zusammensetzung nach einem der vorangehenden Ansprüche, wobei das wenigstens eine wasserunlösliche Poly(meth)acrylat ein Copolymer aus Ethylacrylat und Methylacrylat ist; und wobei das wenigstens eine wasserlösliche Poly(meth)acrylat ein Copolymer aus Methylacrylat, Methylmethacrylat und Methacrylsäure ist.

7. Zusammensetzung nach einem der vorangehenden Ansprüche, wobei die Polymermischung (i) 50 - 90 Gew. - %, vorzugsweise 60 - 80 Gew. - %, bevorzugter etwa 70 Gew. - %, bevorzugter etwa 67 Gew. - %, bezogen auf das Trockengewicht der Polymermischung, an wenigstens einem wasserunlöslichen Poly(meth)acrylat und 10 - 40 Gew. - %, vorzugsweise 20 - 30 Gew. - %, bevorzugter etwa 23 Gew. - %, bezogen auf das Trockengewicht der Polymermischung, an wenigstens einem wasserlöslichen Poly(meth)acrylat umfasst.

8. Zusammensetzung nach einem der vorangehenden Ansprüche, wobei das nicht-poröse inerte Gleitmittel Stearat, vorzugsweise Mg-Stearat, oder Talkum ist.

9. Zusammensetzung nach Anspruch 8, die 5 - 30, vorzugsweise 8 - 15, bevorzugt etwa 9 Gew. - %, bezogen auf das Trockengewicht der Polymermischung von (i), an Mg-Stearat umfasst.

10. Zusammensetzung nach Anspruch 8, die 100 bis 300, vorzugsweise 150 bis 250, bevorzugt etwa 200 Gew. - %, bezogen auf das Trockengewicht der Polymermischung von (i), an Talkum umfasst.

11. Zusammensetzung nach einem der vorangehenden Ansprüche, die weiter eine Sperrschicht umfasst, die wenigstens ein Poly(meth)acrylat und ein nicht-poröses Gleitmittel, ausgewählt aus der Gruppe, bestehend aus Stearaten, Kaolin, Pharmaglas, Talkum und Mischungen davon, umfasst, wobei das wenigstens eine Poly(meth)acrylat vorzugsweise ein wasserlösliches Poly(meth)acrylat ist und das nicht-poröse Gleitmittel vorzugsweise ein Stearat ist.

12. Zusammensetzung nach einem der Ansprüche 1 bis 11 für eine einmal tägliche Verabreichung.

13. Zusammensetzung nach einem der Ansprüche 1 bis 11 für eine einmal tägliche Verabreichung zur Erzielung einer stetigen 24-stündigen oder längeren Absorption des wenigstens einen pharmazeutischen Wirkstoffes.

## Revendications

1. Composition pharmaceutique multi-particules, les particules ayant une taille de particule comprise entre 2,2 et 4 mm, de préférence de 2,5 à 3,1 mm et plus préférentiellement d'environ 2,8 mm, chaque particule comprenant :
(a) un noyau contenant ou portant au moins un agent pharmaceutiquement actif ;
(b) au moins une couche présentant une adhésion et/ou étant écrasable dans le côlon comprenant (i) un ou plusieurs poly(méth)acrylates, et (ii) un lubrifiant inerte non-poreux, choisi dans le groupe constitué de stéarates, kaolin, pharmaglass, talc et des mélanges de ceux-ci ; et
(c) une couche supérieure contenant au moins un agent pharmaceutiquement actif,
dans lequel le ou les poly(méth)acrylates (i) représentent un mélange de polymères comprenant 40 à 95% en poids, sur la base du poids sec du mélange de polymères, d'au moins un poly(méth)acrylate insoluble dans l'eau et 5 à 60% en poids, sur la base du poids sec du mélange de polymères, d'au moins un poly(méth)acrylate soluble dans l'eau.

2. Composition de la revendication 1, dans laquelle la couche supérieure contient l'agent pharmaceutiquement actif ou au moins l'un des agents pharmaceutiquement actifs du noyau (a).

3. Composition de la revendication 1 ou 2, dans laquelle l'agent pharmaceutiquement actif a une demi-vie d'environ 24 heures ou moins.

4. Composition de l'une quelconque des revendications 1 à 3, dans laquelle l'agent pharmaceutiquement actif est un opioïde.

5. Composition de l'une quelconque des revendications précédentes, dans laquelle la teneur de polymère est d'environ 40% en poids du poids total de la composition.

6. Composition de l'une quelconque des revendications précédentes, dans laquelle l'au moins un poly(méth)acrylate insoluble dans l'eau est un copolymère d`acrylate d'éthyle et d'acrylate de méthyle ; et dans laquelle l'au moins un poly(méth)acrylate soluble dans l'eau est un copolymère d'acrylate de méthyle, de méthacrylate de méthyle et d'acide méthacrylique.

7. Composition de l'une quelconque des revendications précédentes, dans laquelle le mélange de polymères (i) comprend
de 50 à 90% en poids, de préférence de 60 à 80% en poids, plus préférentiellement environ 70% en poids, plus préférentiellement environ 67% en poids, sur la base du poids sec du mélange de polymères, d'au moins un poly(méth)acrylate insoluble dans l'eau, et de 10 à 40% en poids, de préférence de 20 à 30% en poids, plus préférentiellement environ 23% en poids, sur la base du poids sec du mélange de polymères, d'au moins un poly(méth)acrylate soluble dans l'eau.

8. Composition de l'une quelconque des revendications précédentes, dans laquelle le lubrifiant inerte non poreux est le stéarate, de préférence le stéarate de Mg, ou le talc.

9. Composition de la revendication 8, comprenant de 5 à 30%, de préférence de 8 à 15%, plus préférentiellement environ 9% en poids, sur la base du poids sec du mélange de polymères de (i), de stéarate de Mg.

10. Composition de la revendication 8, comprenant de 100 à 300%, de préférence de 150 à 250%, plus préférentiellement environ 200% en poids, sur la base du poids sec du mélange de polymères de (i), de talc.

11. Composition de l'une quelconque des revendications précédentes, comprenant en outre une couche barrière comprenant au moins un poly(méth)acrylate et un lubrifiant non-poreux, choisis dans le groupe constitué de stéarates, kaolin, pharmaglass, talc et des mélanges de ceux-ci,
dans laquelle l'au moins un poly(méth)acrylate est de préférence un poly(méth)acrylate soluble dans l'eau et le lubrifiant non-poreux est de préférence un stéarate.

12. Composition de l'une quelconque des revendications 1 à 11 pour une administration une fois par jour.

13. Composition selon l'une quelconque des revendications 1 à 11 pour une administration une fois par jour destinée à obtenir une absorption régulière de 24 heures ou plus de l'au moins un agent pharmaceutiquement actif.
